Europäisches Patentamt
European Patent Office
Office européen des brevets

(19)

(11) Publication number: **0 385 464 A1**

# EUROPEAN PATENT APPLICATION

(12)

(21) Application number: **90103988.3**

(51) Int. Cl.[5]: **A61K 31/72**

(22) Date of filing: **01.03.90**

(30) Priority: **03.03.89 JP 49899/89**

(43) Date of publication of application: **05.09.90 Bulletin 90/36**

(84) Designated Contracting States: **CH DE ES FR GB IT LI**

(71) Applicant: **SAPPORO BREWERIES LIMITED**
**No. 10-1, Ginza 7-chome Chuo-ku**
**Tokyo(JP)**

(72) Inventor: **Ito, Masahiko**
**74-1 Shichishanomiya, Watari**
**Fukushima-shi, Fukushima-ken(JP)**
Inventor: **Yamamoto, Hisao, c/o SAPPORO BREWERIES LIMITED**
**Pharmaceutical Research Lab., 10, Okatohme**
**Yaizu-Shi,, Shizuoka-ken(JP)**
Inventor: **Kobayashi, Fujio, c/o SAPPORO BREWERIES LIMITED**
**Pharmaceutical Research Lab., 10, Okatohme**
**Yaizu-Shi,, Shizuoka-ken(JP)**
Inventor: **Uchida, Kiichi, c/o SAPPORO BREWERIES LIMITED**
**Research & Development Div., 10-1, 7-chome, Ginza**
**Chuo-ku, Tokyo(JP)**

(74) Representative: **Türk, Gille, Hrabal**
**Brucknerstrasse 20**
**D-4000 Düsseldorf 13(DE)**

## (54) Compositions having antiviral and immunoactivating effects.

(57) A composition having an antiviral effect in combination with an immunoactivating effect, which contains as an effective ingredient a sulfate of an immunoactive polysaccharide RON having a specific basic skeleton.

*FIG. 1*

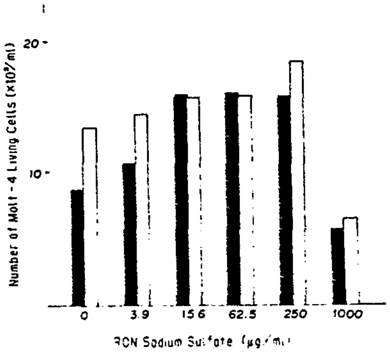


EP 0 385 464 A1

Xerox Copy Centre

# COMPOSITIONS HAVING ANTIVIRAL AND IMMUNOACTIVATING EFFECTS

## BACKGROUND OF THE INVENTION

The present invention relates to a composition having an antiviral effect in combination with an immunoactivating effect and, more particularly, to a composition as an effective ingredient a sulfate of a specific immunoactive polysaccharide RON.

The acquired immunodeficiency syndrome (hereinafter AIDS for short) is a diseases found in U.S.A. in 1981, which is caused by AIDS viruses (hereinafter HIV for short) belonging to human retroviruses. When infected with such virses, helper T lymphocytes are destroyed to induce cellular immunodeficiency and eventually opportunistic infections, malignant tumors, etc. by various microbes including protozoans, thus causing high mortality rates.

As well-known, HIV exists in the blood, sperm and saliva of an HIV carrier and is transmitted by transfusion or other causes.

Most of medicines now available against AIDS have an action on the inhibition of the activity of reverse transcriptase, as represented by 3-azido-deoxythymidine, suramin and HPA-23. However, due to their strong side effects, such remedies have a disadvantage of being uncapable of being administrated to carriers over an extended period to time so as to make them asymptomatic. For that reason, there is a great need for more effective and less side-effective medicines.

It has also been known that polysaccharide sulfates obtained from the existing polysaccharides such as dextran have an antiviral effect. However, as already pointed out, such starting polysaccharides as dextran have a disadvantage of possessing no immunoactivity and losing their own immunoactivating effect upon sulfated such as β-glucan (Lentinan). Thus, a specific substance capable of meeting both the antiviral and immunoactivating effects at the same time is now demanded as AIDS remedies/preventives. Problems with existing dextran or similar substances are that they are unsuitable for extended administration or administration to hemophiliacs, etc. because of their strong blood coagulating action.

To overcome the above problems, we have made an investigation on the antiviral and immunoactivating effects of a polysaccharide sulfate obtained from a novel polysaccharide RON that we had found immunoactivating effect, and had consequently found that such the polysaccharide sulfate has a considerable effect upon inhibiting the proliferation of HIV without losing its original immunoactivating effect.

More specifically, the present invention is concerned with a composition having an antiviral effect in combination with an immunoactivating effect, which contains as an effective ingredient a sulfate of an immunoactive polysaccharide RON (hereinafter often referred to as the RON sulfate) that is an α-1,6-glucan and containing a small amount of 3,6-branched side chains with the basic skeleton expressed by the following general formula:

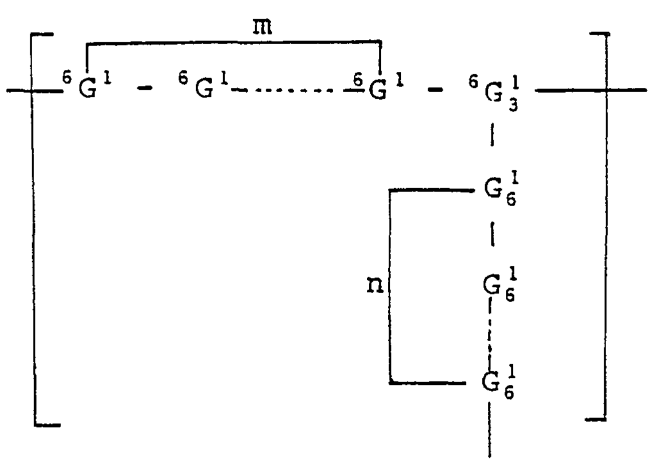

wherein G stands for α-D-glucopyranose and the sum of m and n is an interger of 100 or less.

## BRIEF DESCRIPTION OF THE DRAWING

The present invention will now be explained specifically but not exclusively with reference to the accompanying drawing, in which Figure 1 is a graphical view showing an influence of the sodium sulfate of RON on Molt-4 live cells.

## DETAILED EXPLANATION OF THE INVENTION

The polysaccharide RON used in the present invention may be prepared by such methods as set forth in Japanese Patent Kokai Publication No. 53220/1986 and Japanese Patent Application Nos. 29381/1989, 29382/1989 and 29383/1989 specifications. This substance is an $\alpha$-1,6-glucan and containing a small amount of 3,6-branched side chains as expressed by the above general formula.

The sulfation of the polysaccharide RON may be carried out in conventional manners. If possible, the reaction should more preferably be carried out at high temperature than at low temperature. The obtained RON sulfate may be used intact as the effective ingredient in the present invention, because it is acid-decomposed into a low molecule during the reaction. However, preference is given to a low-molecular fraction of the RON sulfate obtained by such operations as ultrafiltration and having a molecular weight of 10,000 to 100,000 inclusive. It is noted that by the sulfation of the RON substance, 1 to 3, preferably 2 to 3 sulfate groups should be introduced per $\alpha$-D-glucopyranosyl residue. If required, this RON sulfate may be used in the form of such a pharmaceutically allowable salt as sodium salt, or as medicines in combination with suitable carriers or vehicles.

The compositions containing the RON sulfate as an effective ingredient may be administrated in the form of any one of injections, oral preparations, suppositories and the like.

Although varying depending upon the conditions of patients, what for, how to administrate, age and other factors, the composition may usually and suitably be administrated at a dosage of 1 to 500 mg/kg by way of an oral route.

Required for the remediation of AIDS is a pharmaceutical preparation meeting both the anti-HIV effect that deactivates HIV and the immunoactivating effect that prevents a reduction of immunological competence by HIV. In this connection, the sodium sulfate of RON according to the present invention is not only effective for the treatment of AIDS patients but has also an advantage of being administrable over an extended period of time to make HIV carriers asymptomatic, because it has an antiviral effect such as anti-HIV activity in combination with an immunoactivating effect, is administrable by way of an oral route and is less anticoagulant. Thus, the present RON sulfates are far much effective as remedies for making AIDS asymptomatic.

The present invention will now be explained with reference to the following examples.

### Preparative Example 1 - Preparation of Sodium Sulfate of RON

A mixture of 2.0 g of RON, 5 g of a sulfur trioxide/trimethylamine combination (made by Aldrich Co., Ltd.) and 50 ml of dimethyl sulfoxide was stirred at 120 °C for 2 hours, then cooled down to 50 °C and finally stirred for another 24 hours. After cooling down to room temperature, 50 ml of a 10% solution of sodium hydroxide was added to the resulting solution, which was then dialyzed in a water stream in Spector/por 2 (made by Spectrum Medical Industries Co., Ltd.) for 2 days. The liquid content was concentrated under reduced pressure to 50 ml and freeze-dried to give 3.1 g of a pale brown, powdery product, which was found to have the following physicochemical properties.

| Elemental analysis - for $(C_6H_8S_2O_{11}Na_2)n$ | | | | |
|---|---|---|---|---|
| | C | H | S | Na |
| Calculated: | 19.67 | 2.20 | 17.51 | 12.55 |
| Found: | 19.40 | 2.11 | 17.69 | 12.71 |

Optical Rotation:
$[\alpha]_D^{25}$ = +63.3 (C = 0.15, $H_2O$)
$^{13}C$-NMR (22.5 MHz)
96.60 (C-1), 78.72-74.50 ($\underline{C}$-O-$SO_3Na$)

Preparative Example 2

An aqueous solution of 1.0 g of the sodium sulfate of RON obtained in Preparative Example 1 dissolved in 20 ml of water was subjected to ultrafiltration with a fleaker (Spectra Por/4 made by Spectrum Medical Industries Co., Ltd.), and the filtrate was then washed twice with 10 ml of water. The combined filtrate and washings were freeze-dried to give 357 mg of a low-molecular fraction of the sodium sulfate of RON (having a molecular weight of 10,000 to 100,000) in the form of a pale brown, powdery product.

On the other hand, the residues were dissolved in 20 ml of water, and the resulting solution was then freeze-dried to give 582 mg of a high-molecular fraction of the sodium sulfate of RON (having a molecular weight of more 100,000 in the form of a pale brown, powdery product.

Preparative Example 3

A suspension of 4.0 g of RON in 100 ml of dimethyl sulfoxide was allowed to react with 25 g of a sulfur trioxide pyridine complex (made by Aldrich Co., Ltd.) under agitation at -15°C for 7 days. The reaction solution was put in a dialysis membrane (24 Å, made by Union Carbide Co., Ltd.), in which it was dialyzed in a 5% sodium bicarbonate solution for 12 hours and in a water stream for 2 days. The dialyzate was then concentrated to 50 ml and freeze-dried to give 6.3 g of the sodium sulfate of RON in the form of white powders.

Example 1

Investigations were made on whether or not the sodium sulfate of RON had an effect upon inhibiting the proliferation of HIV, as stated below. The cell line used was Molt-4 clone No. 8, and the viruses used were obtained from a Molt-4/HTLV-III producing strain.

$2 \times 10^5$ Molt-4 cells were infected with $6 \times 10^2$ HIV viruses, and were then cultured in an RPMI 1640 liquid medium containing antibiotics and 10% of the serum of a bovine embryo with the addition of varied concentrations of the low-molecular fraction of the sodium sulfate of RON. Subsequently, the above cultured liquid was cultured for a further 2 days with the addition of varied concentrations of the above low-molecular fraction of the sodium sulfate of RON. On the other hand, uninfected Molt-4 cells were likewise cultured as control.

After the completion of culture, the number of live cells was determined by the trypan blue technique to assay an influence of the sodium sulfate of RON. The results are set forth in Figure 1, wherein a white bar stands for cells uninfected with HIV and a black bar indicates cells infected with HIV. As clearly understood from Fig. 1, the low-molecular fraction of the sodium sulfate of RON exerts an inhibitory effect at a concentration of 15.6 to 250 μg/ml upon the denaturation of the HIV-infected cells. On the other hand, the effect of the low-molecular fraction of the sodium sulfate of RON upon the denaturation of the cells uninfected with HIV is observed at 1 mg/ml.

Example 2

In order to examine the immunoactivating effect of the sodium sulfate of RON, the activity of a reticuloendothelial system in mouse was assayed as an index to the capability of preying upon carbon granules under the following conditions.

Drugs Under Test

To this end, use was made of the sodium sulfate of RON obtained in Preparative Example 1, the low- and high-molecular fractions of the sodium sulfate of RON obtained in Preparative Example 2 and RON.

Animals Under Test

ICR mice (male, 6-week age) were pre-fed for 1 week and then tested at the age of 7 weeks. During the

administration of the drugs, the animals weighed 31.19 ±1.07 g on the average.

Determination of the Capability of Preying on Carbon Granules

The drugs under test were dissolved in phosphate buffer saline (PBS, pH 7.2) and intraperitoneally administrated to the mice once (at a dosage of 0.1 ml/10 g of body weight).

Four days after the administration of the drugs, Fount India Ink (made by Pelican Co., Ltd.) was diluted five times with PBS and injected into the caudal veins at 0.08 ml/10 g of body weight.

After 30 seconds and 10 minutes, 20 µl of blood were gathered from the plexuses of the supraorbital veins, and were then dissolved in 1.98 ml of a 0.1% solution of $Na_2CO_3$ to determine the absorbent at a wavelength of 675 nm with a spectrophotometer (Spectrophotometer Model U-3200 made by Hitachi Co., Ltd.)

The Granulopectic index (K value) was found by the following equation established by Biozzi et al (in 1953).

$$K = \frac{\log C_1 - \log C_2}{T_1 - T_2} = \frac{\log (OD_1 - b) - \log (OD_2 - b)}{T_1 - T_2}$$

wherein C is the concentration of carbon,
T is the blood gathering time (min.),
OD is the absorbent (found), and
b is the absorbent of the standard blood diluted 100 times.

The results of the sodium sulfate of RON (Preparative Example 1) are set out in Table 1, from which it is found that the sodium sulfate of RON shows as strong a carbon granule preying capability as RON.

Table 1

| Specimen (mg/kg) | Number of Animals | K value | Ratio to Control | Significant Difference from Control |
|---|---|---|---|---|
| Control | 8 | 2.72±0.52 | 1.00 | |
| RON, 30 mg/kg | 10 | 4.41±0.44 | 1.62 | p<0.001 |
| Sodium Sulfate of RON, 30 mg/kg | 10 | 3.51±0.53 | 1.29 | p<0.01 |
| Sodium Sulfate of RON, 100 mg/kg | 9 | 4.01±0.61 | 1.48 | p<0.001 |

Thus, the sodium sulfate of RON was also found to have an increased effect on the activity of the reticuloendothelial system. Next, investigations were made of how much influence a difference in the molecular weight of the sodium sulfate of RON produced. The results are shown in Table 2, which reveals that stronger carbon granule preying capability is found with the low-molecular fraction of the sodium sulfate of RON than with the high-molecular one.

Table 2

| Specimen (mg/kg) | Number of Animals | K value | Ratio to Control | Significant Difference from Control |
|---|---|---|---|---|
| Control | 7 | 2.58±0.44 | | |
| RON, 30 mg/kg | 8 | 4.12±1.25 | 1.60 | $p<0.001$ |
| Sodium Sulfate of RON-1*, 100 mg/kg | 8 | 3.48±0.86 | 1.35 | $p<0.05$ |
| Sodium Sulfate of RON-2**, 100 mg/kg | 8 | 4.10±0.81 | 1.59 | $p<0.01$ |
| Sodium Sulfate of RON-3***, 100 mg/kg | 9 | 2.97±0.97 | 1.15 | |

*Sodium Sulfate of RON-1: obtained in Preparative Example 1
**Sodium Sulfate of RON-2: low-molecular fraction of Preparative Example 2
***Sodium Sulfate of RON-3: high-molecular fraction of Preparative Example 3

## Claims

1. A composition having an antiviral effect in combination with an immunoactivating effect, which contains as an effective ingredient a sulfate of an immunoactive polysaccharide RON having a basic skeleton expressed by the following general formula:

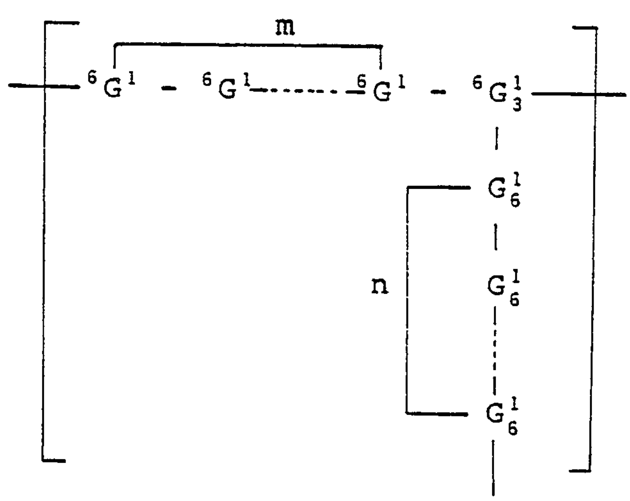

wherein G stands for α-D-glucopyranose and the sum of m and n is an integer of 100 or less.

2. A composition as claimed in Claim 1, wherein the sulfate of an immunoactive polysaccharide RON includes 1 to 3 sulfate groups introduced per α-D-glucopyranose.

FIG.1

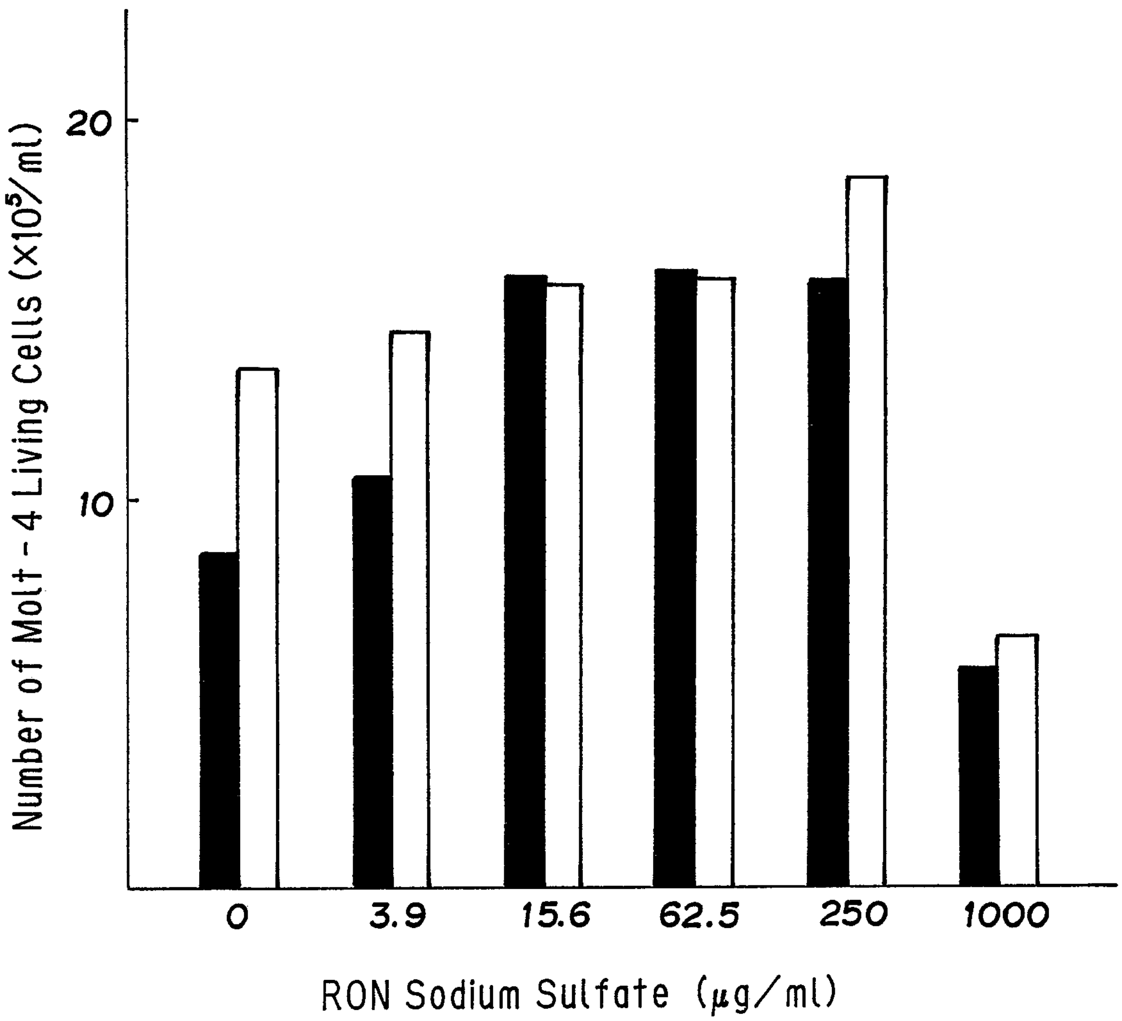
Number of Molt-4 Living Cells (×10⁵/ml)
20
10
0
3.9
15.6
62.5
250
1000
RON Sodium Sulfate (μg/ml)

European Patent Office

# EUROPEAN SEARCH REPORT

Application Number

EP 90 10 3988

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 172 559 (SAPPORO BREWERIES LTD) * Abstract * --- | 1 | A 61 K 31/72 |
| Y | CHEMICAL ABSTRACTS, vol. 109, no. 19, 7th November 1988, page 14, abstract no. 162907x, Columbus, Ohio, US; K. MIZUMOTO et al.: "Sulfated homopolysaccharides with immunodulating activities are more potent anti-HTLV-III agents than sulfated heteropolysaccharides", & JPN. J. EXP. MED. 1988, 58(3), 145-51 * Abstract * --- | 1 | |
| A | US-A-4 614 733 (C. YOSHIKUMI) * Column 14, lines 61-67 * --- | | |
| A | CHEMICAL ABSTRACTS, vol. 96, no. 11, 15th March 1982, page 28, abstract no. 79475x, Columbus, Ohio, US; Y. HIYAMA et al.: "Antiviral activity of schizophyllan (SPG), an antitumor polysaccharide", & KINKI DAIGAKU IGAKU ZASSHI 1981, 6(3), 387-91 * Abstract * ----- | | |
| | | | TECHNICAL FIELDS SEARCHED (Int. Cl.5) |
| | | | A 61 K<br>C 08 B |

The present search report has been drawn up for all claims

| Place of search | Date of completion of the search | Examiner |
|---|---|---|
| THE HAGUE | 28-05-1990 | SOMERVILLE F.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)